# EUROPEAN PATENT APPLICATION

(11) **EP 1 563 842 A1**
(43) Date of publication of application: **17.08.2005**
(21) Application number: 04002296.4
(22) Date of filing: 03.02.2004
(51) Int. Cl.: A61K 35/78, A61P 1/00

(54) **Simarouba amara and/or Momordica charantia extracts for the treatment of coccidiosis in poultry**

(71) Applicant: Novartis AG, 4056 Basel (CH)
(72) Inventor: The designation of the inventor has not yet been filed
(74) Representative: Gros, Florent

(57) **Abstract**

Described is the use of natural ingredients *Simarouba amara* dried bark or an extract thereof and low concentrations of *Momordica charantia* fruit extract either alone or in combination with each other in the resistance management and control of coccidiosis in poultry. It further relates to veterinary compositions containing said natural ingredients, the preparation of these veterinary compositions, a method of controlling coccidiosis in poultry comprising the administration of said natural ingredients or said veterinary compositions to poultry, and the use of said natural ingredients for the preparation of these veterinary compositions or for treating coccidial infections in poultry. The invention also comprises the use of said natural ingredients as an additive to dry or wet poultry food or drinking water for the prophylactic or curative treatment of coccidial infections in poultry.

## Description

This invention relates to the use of natural ingredients *Simarouba amara* dried bark or an extract thereof and low concentrations of *Momordica charantia* fruit extract either alone or in combination with each other in the resistance management and control of coccidiosis in poultry. It further relates to veterinary compositions containing said natural ingredients, the preparation of these veterinary compositions, a method of controlling coccidiosis in poultry comprising the administration of said natural ingredients or said veterinary compositions to poultry, and the use of said natural ingredients for the preparation of these veterinary compositions or for treating coccidial infections in poultry. The invention also comprises the use of said natural ingredients as an additive to dry or wet poultry food or drinking water for the prophylactic or curative treatment of coccidial infections in poultry.

One of the most important diseases in poultry is coccidiosis because it meanwhile is spread over all continents and causes heavy economic losses to the poultry industry.

Coccidiosis causes severe pathology in the intestines and ceca of poultry. It affects mainly the upper, middle, and lower small intestine, the rectum, the colon, and the ceca. Coccidiosis is caused by infections with protozoans (*apicomplexa*) of the genus *Eimeria* (called coccidia). The common problem causing species in poultry (chickens, turkeys) are *Eimeria acervulina, E. brunett, E. maxima, E. mivati, E. necatrix, E. tenella, E*. *adenoeides, E. dispersa, E*. *gallopavonis, E. meleagridis, E. aythyae*, and E. *burcephalae*. The habitats of the *Eimeria* in avian, depends on the species of *Eimeria* and the poultry species. The coccidian can habitate the upper, middle, and lower small intestine, the rectum, the colon, the kidneys, and the ceca.

In context with the present invention the expression poultry is used for all sort of breeds of domesticated birds independent of their age, comprising chickens, ducks, geese, fowl, pigeons, turkeys, and ostriches. Said expression is preferably used for chickens, ducks, geese, and turkeys, and most preferably for chickens.

Birds become infected usually by ingesting water, feed or bedding material contaminated with the infectious stage, i.e. sporulated oocyst. In the gut, the oocyst wall is mechanically and/or enzymatically broken. Sporocysts are set free and exposed to enzymes, which releases sporozites. The sporozites move actively and enter epithelial cells, where they develop further. Intracellularly, the sporozite develops into a 1^{st} generation schizont. In the schizont, 100s-1000s of merozites form. The host cell is destroyed and the merozites are released to invade other epithelial cells and form 2^{nd} generation schizonts and more merozites. Some *Eimeria spp.* repeat the cycle to form 3^{rd} generation schizonts. The trigger for switching to sexual developmental stages is unknown.

However, when triggered, macrogametes and microgametes are formed. Microgametes fertilize macrogametes leading to the formation of intracellular zygotes. The zygote becomes an oocyst, destroying the host cell, and leaves the animal via the faeces. The unsporulated oocyst sporulates upon exposure to warm and wet soil or litter, thus becoming the infective life stage. The invasion and death of the epithelial cells leads to the disease of coccidiosis.

The disease is manifested by hemorrhage, accumulation of blood in the ceca, passage of blood in the droppings, weakness and digestive disturbances. The disease often terminates in the death of the animal, but the fowl which survive severe infections have had their market value substantially reduced as a result of the infection. Coccidiosis is, therefore, a disease of great economic importance and extensive work has been done to find new and improved methods for controlling and treating coccidial infections in poultry.

In the poultry industry it is common practice to include anticoccidial agents in poultry feed for most of the bird's life to control or prevent coccidiosis outbreak. Current control methods rely on the use of either coccidiostats (amprolium, clopidol, halofuginone, lasalocid, monensin, nicarbazine, nitrofurazone, nitromide, sulfadimethoxine, sulfaquinoxyline, salinomycin, zoalene) or vaccines. Live virulent, live attenuated, and recombinant vaccines are being researched. While there are some vaccines on the market, problems associated with these include, specificity of the immune response, availability, the need to recycle the vaccine to ensure continued efficacy. Vaccines primarily play a role in breeding birds.

Coccidiostats are typically added to the feed or water at rates of 3 to 250 ppm for 3 to 6 days or longer.

However, treatment with coccidiostats has severe disadvantages.

For all coccidiostats, there is a withdrawal time before slaughter, and most are not permitted to be used in birds for egg production. This withdrawal time guarantees that the meat that is foreseen for consumption does not contain drug residues that might affect the health of the consumer. For each product with marketing approval the withdrawal time is defined by the authority in each country and must be indicated on the product label. The dosage, length of treatment time and withdrawal time should be followed exactly. Failure to do so may affect the drug withdrawal time and lead to illegal drug residues. Increasing dosage or treatment times may also result in unwanted or harmful side effects, or even death, in the birds.

Products without withdrawal time have clear competitive advantage in the poultry market.

Coccidiostats do not kill the pathogen, but interrupt the development of specific life stages, for example, by intervening with the production of a specific protein so that the pathogen cannot properly develop. With this interaction on a specific point they decrease the adverse impacts of the *Eimeria* on the intestine and ceca, giving the poultry's immune system time to build up and fight the infections. Coccidiostats are typically added to the feed or water at rates of 3 to 250 ppm for 3 to 6 days or longer.

Coccidiostats fall under different chemical types (e.g., ionophores, antibiotics, and sulfonamides). Dependent on the chemical class and the exact compound, the mode of action or life stage interrupted varies. For example, clopidol acts against the sporozoite stage, allowing host cell penetration without development. Halofuginone is effective against asexual stages. The mechanism of action is not known.

The major disadvantage for all of the chemical coccidiostats is, there is a risk that the causative organisms will rapidly develop resistance after continuous or repeated exposure to any particular drug. Furthermore, conventionally used anticoccidial agents such as sulfanilamides, nitrofurans, quinolines, antithiamines, benzoamides, and polyether-based antibiotics are often toxic to the hosts. Therefore, there is a continuing need to identify new anticoccidial compounds, which are either chemically completely new or which consists of combinations with which decrease the rate of resistance development and/or control multiresistant parasite strains.

Lastly, there are health and safety concerns with some of these coccidiostats because of the potential overuse of antibiotics and cross-resistance. Therefore, not all of them will be available in the future, world-wide. Currently, several coccidiostats are already banned in the European Union, including amprolium and meticlorpindol.

This is why there is a clear need for improved and safer products and especially for products that do not trigger the development of resistance. The development of resistance against chemicals is a severe problem in monocellular eukaryotic organisms. The major reason for this is because such organisms replicate very fast and can rapidly adapt their metabolism to environmental changes. This is especially true for the protozoans that cause coccidiosis in poultry, where the chemicals are administered prophylactically all over the year, and the pathogen is permanently confronted with the chemical. In such an environment resistance can develop very fast.

It has now surprisingly been found that two natural ingredients, when used within specific dose ranges, act as coccidiostats and have the same type of impact on decreasing damage in the poultry and on production. Specifically, intestinal and cecal lesions caused by coccidia are decreased and this decrease in coccidial damage improves weight gain and feed efficiency compared to poultry with untreated coccidia infections.

The natural ingredients of the present invention can also be applied in the form of a poultry feed supplement or in form of medicated poultry feed. Thus, the present invention also relates to such a poultry feed supplement or medicated poultry feed, and the use of *Simarouba amara* dried bark or an extract thereof and the use of *Momordica charantia* fruit extract either alone or in combination with each other for the manufacture of said a poultry feed supplement or medicated poultry feed.

The veterinary compositions of the present invention are preferably oral compositions, which exhibit a coccidiostatic activity and promote feed efficiency and weight gain in poultry, and which are intended in particular for reducing the impact of *Eimeria spp.* infections in poultry.

Therefore, an important objective of the present invention is to provide a means for preventing the development of resistant *Eimeria* strains that cause coccidiosis in poultry comprising rotation of the administration of a coccidiostatically effective amount of *Simarouba amara* dried bark or an extract thereof and of a coccidiostatically effective amount of *Momordica charantia* fruit extract or alternatively the administration of a coccidiostatically effective amount of a combination of *Simarouba amara* dried bark or an extract thereof and *Momordica charantia* fruit extract together with one or more carriers that are physiologically well-tolerated by birds.

Even more preferred is providing a resistance management tool wherein during one period of time *Simarouba amara* and the next season *Momordica charantia* is administered or alternatively a resistance management tool wherein a combination of *Simarouba amara* and *Momordica charantia* is administered.

*Simarouba amara* and *Momordica charantia* provide a unique opportunity for managing resistance, because both contain several different compounds that can be active as coccidiostats, thus either one is like administering a combination of several compounds with additive and/or complementary activity. With single compounds, resistance is most likely to develop. The chemistry of *Simarouba amara* and *Momordica charantia* differ from each other, so when used in combination or rotation even more diversity of activity is present.
Specifically, these can be used in rotation by changing which is used every 4-6 months within a facility (e.g., every 4^{th} flock change). Alternatively, one can be used during the starter growth phase of the poultry and the other used during the finishing period.

Thus, the present invention relates essentially to a veterinary composition and their use for the control of coccidiosis in poultry comprising a coccidiostatically effective amount of *Simarouba amara* dried bark or an extract thereof or of *Momordica charantia* fruit extract or of a coccidiostatically effective amount of a combination of *Simarouba amara* dried bark or an extract thereof and *Momordica charantia* fruit extract together with one or more carriers that are well-tolerated by birds.

### Preferred embodiments or the present invention are amongst other:

Veterinary compositions as described above wherein the active ingredient is *Simarouba amara* dried bark or an extract thereof.
Veterinary compositions as described above wherein the active ingredient is *Momordica charantia* fruit extract.

Veterinary compositions as described above wherein the active ingredient is a combination of *Simarouba amara* dried bark or an extract thereof and *Momordica charantia* fruit extract. This combination product is especially preferred due to its extraordinary efficacy, tolerability and its ability to prevent resistance development.

### Equally preferred is:

The use of *Simarouba amara* dried bark or an extract thereof or of *Momordica charantia* fruit extract or of a coccidiostatically effective amount of a combination of *Simarouba amara* dried bark or an extract thereof and *Momordica charantia* fruit extract for the preparation of a veterinary composition for the control of coccidiosis in poultry.
The use of *Simarouba amara* dried bark or an extract thereof for the preparation of a veterinary composition for the control of coccidiosis in poultry.
The use of *Momordica charantia* fruit extract for the preparation of a veterinary composition for the control of coccidiosis in poultry.
The use of a combination of *Simarouba amara* dried bark or an extract thereof and *Momordica charantia* fruit extract for the preparation of a veterinary composition for the control of coccidiosis in poultry.

### Further preferred embodiments are:

A method for controlling coccidiosis in poultry comprising the administration of a coccidiostatically effective amount of *Simarouba amara* dried bark or an extract thereof or of *Momordica charantia* fruit extract or of a coccidiostatically effective amount of a combination of *Simarouba amara* dried bark or an extract thereof and *Momordica charantia* fruit extract together with one or more physiologically acceptable carriers to a bird suffering from a coccidial infection.
The method as characterized above wherein the active ingredient is *Momordica charantia* fruit extract.
The method as characterized above wherein the active ingredient is *Momordica charantia* fruit extract.
The method as characterized above wherein the active ingredient is a combination of *Simarouba amara* dried bark or an extract thereof and *Momordica charantia* fruit extract.

Equally preferred is the use of a coccidiostatically effective amount of *Simarouba amara* dried bark or an extract thereof or of *Momordica charantia* fruit extract or of a coccidiostatically effective amount of a combination of *Simarouba amara* dried bark or an extract thereof and *Momordica charantia* fruit extract in a method for treating coccidial infections in poultry.

Equally preferred in said method for treating coccidial infections in poultry is the use of a coccidiostatically effective amount of *Simarouba amara* dried bark or an extract thereof.
Equally preferred in said method for treating coccidial infections in poultry is the use of a coccidiostatically effective amount of *Momordica charantia* fruit extract.
Especially preferred in said method for treating coccidial infections in poultry is the use of a coccidiostatically effective amount of a combination of *Simarouba amara* dried bark or an extract thereof and *Momordica charantia* fruit extract.

A specially preferred embodiment of the present invention is a poultry feed supplement or medicated poultry feed for the control of coccidiosis in poultry comprising besides ground dry vegetable- and/or animal-based poultry feed, with or without additives such as proteins, vitamins and minerals, a coccidiostatically effective amount of *Simarouba amara* dried bark or an extract thereof or of *Momordica charantia* fruit extract or of a coccidiostatically effective amount of a combination of *Simarouba amara* dried bark or an extract thereof and *Momordica charantia* fruit extract.

Thus, said poultry feed supplement or medicated poultry feed contains either one or the other of the natural ingredients or a combination of both. Each of said embodiments is equally preferred.

Advantageously said a poultry feed supplement or medicated poultry feed consists of pellets that are usually produced by feed mills. Ground cereal is generally used as base. To that base there are added further constituents such as oil and vegetable and animal proteins. All the constituents are intimately mixed together in a grinding or mixing apparatus, sprayed with water or treated with steam and, at elevated temperature, extruded, i.e. pressed, through a round nozzle having a diameter of about from 2 to 15 mm. During that pressing process, the moistened material is compacted and it leaves the nozzle in the form of a relatively hard bar, which at the nozzle outlet is cut into pieces of desired length, for example about from 5 to 25 mm in length, using a cutting apparatus. The resulting pellets, which are still warm, dry in the air as they are transported away or are taken on a conveyor belt through a heating chamber and dried at about from 80 to 120°C. The finished pellets are rod-shaped or cylindrical; they have a relatively smooth surface and are readily pourable without crumbling or forming dust. They generally have a density of about 1.2 g/cm3.

In general, the procedure for preparation of the medicated feed pellets according to the invention is exactly as for preparation of normal feed pellets. However, before the pressing process the natural active ingredients and the organic, ground and homogenised feed constituents are intimately mixed together, moistened with about from 5 to 10 % by weight of water or steam and compressed into feed pellets at elevated temperatures of about from 60 to 80°C, preferably from 65 to 75°C. Good homogenization is most advantageously achieved when the natural active ingredients are first intimately mixed together with a relatively small portion of the rest of the feed components, as a result of which a so-called premix having a relatively high proportion of natural active ingredients is obtained. A portion of that premix is then mixed together with further feed material to form a further partial mix and that partial mix is, in a final step, diluted to the final concentration with additional feed material. That dilution results in an especially uniform distribution of the encapsulated active ingredient in the pellets.

The pellets are allowed to cool to room temperature and are packed in paper sacks or other suitable containers for storage or for transportation to the end consumer. No special precautionary measures are necessary because the pellets are extremely storage-stable and comprise the active ingredient in a coated foam which shields the active ingredient from environmental influences. No active ingredient comes through to the outside from these storable pellets.

However, it goes without saying that the natural active ingredients or the veterinary compositions according to the present invention can be simply admixed with normal dry or wet poultry food or drinking water.

The herbs involved in the present invention have a long history of use in the treatment of bacteria, viruses and fungi but not for the treatment of coccidial infections in birds. This historical use confirms the safety of the plant material.

Standardized extracts and plant material of *Simarouba amara* and *Momordica charantia* are commercially available.

*Simarouba* is a medium-sized tree that grows up to 20 m high, with a trunk 50 to 80 cm in diameter. It produces bright green leaves 20 to 50 cm in length, small white flowers, and small red fruits. It is indigenous to the Amazon rainforest and other tropical areas in Mexico, Cuba, Haiti, Jamaica, and Central America (Family: *Simaroubaceae;* Genus: *Simarouba;* Species: *amara*). The plant material contains tiny amounts of a wide range of different chemicals, for example, ailanthinone, benzoquinone, canthin, dehydroglaucarubinone, glaucarubine, glaucarubolone, glaucarubinone, holacanthone, melianone, simaroubidin, simarolide, simarubin, simarubolide, sitosterol, tirucalla, etc.

The *Simarouba* plants are known to have similar properties (see the book by Mrs M. GRIEVE entitled "Modern Herbal", edited by Mrs C. F. LEYEL, published by PENGUIN BOOKS, pages 741-742). *Simarouba amara* Aubl., in particular, is a fairly widespread tree in Guiana, where the bark is used by the natives as a remedy for treating dysentery, improving the tonicity of the intestine, promoting intestinal secretions and predisposing the patient to sleep. *Simarouba* is also used in the cosmetic industry in the form of extracts from bark, wood or leaves. US-5,676,948 describes positive dermatological effects such as a significant skin depigmentation activity, enhancement of the protective function of the skin, enhancement of the water barrier function, and keratinocyte differentiation activity. Further activities of *Simarouba* are described in Chemical Abstracts: Chemical Abstracts 110(02)013432 describes the use of *Simarouba amara* stalk extract for demonstrating an antiamebic activity in vitro. Likewise, Chemical Abstracts 109(101)000285 describes a *Simarouba amara* fruit extract for the preparation of an antimalarial drug. Bark and extracts from *Simarouba* are commercially available or can be prepared as along the procedures described in the literature, for example, the production of aqueous and alcoholic extracts from *Simarouba* bark is disclosed in US-5,676,948.

*Momordica charantia*, a member of the *Cucurbitaceae* family is a tropical plant (Family: Cucurbitaceae; Genus: Momordica; Species: charantia). It is widely known as bitter melon. *Momordica charantia* grows in tropical areas, including parts of East Africa, Asia, the Caribbean, and South America, where it is used as a food as well as a medicine. It is a green cucumber shaped fruit with gourd-like bumps all over it. It looks like an ugly, light green cucumber. The fruit should be firm, like a cucumber. It tastes very bitter. Although the seeds, leaves, and vines of bitter melon have all been used, the fruit is the safest and most prevalent part of the plant used medicinally. The immature fruits are a good source of vitamin C and provide some vitamin A, phosphorus, and iron. *Momordica charantia* is a fast-growing, trailing or climbing vine with thin stems and tendrils. It is a very popular plant, because *Momordica charantia* has been used by natives for cancer, diabetes and many infectious diseases. The plant material contains tiny amounts of a wide range of different chemicals, for example, alkaloids, charantin, charine, cryptoxanthin, cucurbitins, cucurbitacins, cucurbitanes, cycloartenols, diosgenin, elaeostearic acids, erythrodiol, galacturonic acids, gentisic acid, goyaglycosides, goyasaponins, guanylate cyclase inhibitors, gypsogenin, hydroxytryptamines, karounidiols, lanosterol, lauric acid, linoleic acid, linolenic acid, momorcharasides, momorcharins, momordenol, momordicilin, momordicins, momordicinin, momordicosides, momordin, momordolo, multiflorenol, myristic acid, nerolidol, oleanolic acid, oleic acid, oxalic acid, pentadecans, peptides, petroselinic acid, polypeptides, proteins, ribosome-inactivating proteins, rosmarinic acid, rubixanthin, spinasterol, steroidal glycosides, stigmasta-diols, stigmasterol, taraxerol, trehalose, trypsin inhibitors, uracil, vacine, v-insulin, verbascoside, vicine, zeatin, zeatin riboside, zeaxanthin, and zeinoxanthin.

The fruit, bitter melon, of the plant, *Momordica charantia* L., has been used as a diet for a long period of time in different countries (Family *Cucurbitaceae*). Extracts of the fruit, seeds, and aerial parts also have a long tradition in use as medicine for humans and has been used as an antibiotic, antioxidant, antidiabetic, and hypotensive agent and so on. *In vivo* studies have shown low toxicity in people. Intravaneous injects have been shown to be toxic in some laboratory animal studies. Oral consumption of water and ethanol extracts of the fruit and leaf have been demonstrated to be safe during pregenancy.

Certain activities of *Momordica charantia* are also described in the patent literature. For example, US-6,124,442 and US-6,103,240 describe herbal sweetening and preservative composition comprising licorice extract and mogrosides obtained from plants belonging to *cucurbitaceae* and/or *momordica*. US2002193310 is directed to an orally active fraction of *Momordica charantia* active peptides thereof, and their use in the treatment of diabetes.

B. Hayat et al. describe in 'The Pakistan Vet. J., 16 (4): 1996, pp 164-166' tests dealing with coccidiosis in chicken. The authors compare weight gain and reduction of oocytes in faeces in broiler chicks after prophylactic administration of preparations containing 'bakin' (*Melia azedarach*) and 'karela' (*Momordica charantia*), in comparison with the antibiotic salinomycin. Positive effects were obtained with high concentrations, i.e. 1.5 Kg *Momordica charantia* in powder form / 50 Kg feed. Efficacy of the powder can not be confirmed in the article, because virulence of the *Eimeria* used was not determined, no lesion scores presented, and no rating of fecal condition presented ― all of which are needed to determine that the coccidia caused an infection. In addition, the study cannot be repeated, because verification of the type of "karela" is not presented. It has now surprisingly been found that a significant reduction of the amount of *Momordica charantia* leads to a decrease in lesion scores caused by virulent *Eimeria* and that concentrated levels of *Momordica charantia* are not toxic to birds.

Activity of both plants *Simarouba amara* and *Momordica charantia* against various protozoans infecting people have been demonstrated. However, products active against one species of parasitic protozoan are not necessarily active against other species or genera or that the product is active in other hosts of that species or genera. This can especially be the case in humans and birds, which have different digestive systems. In addition, activity against *Eimeria* species in poultry has not been demonstrated before with *Simarouba amara*.

Therefore, this invention is based on the discovery of the activity of *Simarouba amara* bark (and extracts thereof) and *Momordica charantia* fruit extract against *Eimeria spp.* causing coccidiosis in poultry and the solving of the technical problem of an appropriate composition of the above plants in a coccidiostat formulation.

Now, the inventors of the present invention have been able to observe that *Simarouba amara* bark and *Momordica charantia* fruit extracts, in specific dose ranges, are particularly effective in decreasing the negative impacts of *Eimeria spp.* in poultry, and it has been possible to demonstrate this activity by means of *in vivo* tests, which will be described and exemplified below.

The natural ingredients are formulated for systemic, preferably oral administration to poultry, e.g. via feed or water. They can be formulated along with customary excipients that are physiologically well-tolerated by birds in suitable forms such as capsules, powders, liquids, and suspensions or as described above in the form of a poultry feed supplement or medicated poultry feed.

The quantity of the doses of the ingredients are in terms of ppm in feed or water and can vary depending on the severity of the coccidian infection, use (treatment verus prophylactic), and bird species.

The material of *Momordica charantia* to be used in treatment of coccidia infections and prevention of intestinal and ceca damage caused by coccidia is an extract standardized to either: 10% charantin or >2.5% bitters and must contain momordicin. This standardized material can be obtained with a hot water and alcohol extract solvent mixture (20/80) of the fruit using ratios of extract to plant material of 10:1 to 15:1. The extract is then dried and ready for use. The final *Momordica charantia* dried extract powder must be soluble in water or ethanol. HPLC/UV or gravimetric methods can be used to verify the contents of the compounds in the extract. A suitable source for such extracts are Exotic Naturals, Mumbai, India and Kingherb International, Changchun, China.

The other natural ingredient is the bark of *Simarouba amara*. The bark is dried and ground into a powder. The bark must contain quassinoids (e.g., ailanthinone, glaucarubinone). One source of such standardized plant material is Cielo Herbs, U.S.A.

This invention relates to the use of the two defined natural ingredients - alone or in combination with each other - for the prophylactic or curative control of coccidia infections in poultry, preferably when given at a rate of 10 to 400 ppm, most preferably when admixed to the feed or water. Both natural ingredients have been shown to decrease cecal lesion scores, improve weight gain, and maintain feed efficiency when given to *E. tenella* infected poultry compared to untreated poultry with and without infections.

### Biological Example: Reduction of E. tenella in poultry

The study consisted of 2 steps. In step 1, isolated *E. tenella* were given to chicks to demonstrate the virulence of the strain to be used in step 2. To be considered virulent, the infection needed to cause a significant 25% increase in mortality, a significant 20% reduction in rate of weight gain, and/or a significant 2.5 unit increase in lesion scores over the non-infected control (p = 0.05).

Materials used in Step 1 included:
1. Sporulated oocysts (in water at a concentration of 100,000/ml)
2. Chickens (40 broilers), arrived when 1 day of age, divided into 4 groups of 10
3. Siliconized microfuge tubes (1.5 ml)
4. Pipettes, pipettors, and pipette tips
5. Scales or balance for weighing chickens with check weights for scale verification.

The test inocula and measurements were:
1. Day 12 of age, weigh chickens (group feed consumption measured concurrently with body weight gain)
2. Day 14 of age, weigh chickens
3. Day 14 of age, orally inoculate chickens with preformed inocula
a. Make dilutions of sporulated oocysts so have 6 tubes (one extra of each tube):
   1. 11 tubes: 1 ml inoculum
   2. 11 tubes: 0.1 ml inoculum & 0.9 ml water
   3. 11 tubes: 0.01 ml inoculum & 0.99 ml water
   4. 11 tubes: 1 ml water

4. Day 21 of age, weigh chickens
5. Day 21 of age, euthanatize chickens and perform lesion scores (Johnson & Reid, 1970)

| | | |
|---|---|---|
| a. | 0 | No gross lesions |
| b. | +1 | Very few scattered petechiae on the cecal wall; no thickening of the |
| | | cecal walls; normal cecal contents present. |
| c. | +2 | Lesions more numerous with noticeable blood in the cecal contents, |
| | | cecal wall is somewhat thickened; normal cecal contents present. |
| d. | +3 | Large amounts of blood or cecal cores present; cecal walls greatly |
| | | thickened; little, if any, fecal contents in the ceca. |
| e. | +4 | Cecal wall greatly distended with blood or large caseous cores; fecal |
| | | debris lacking or included in cores. Dead birds scored as +4. |

Calculate weight gain for live and dead birds.
In step 2, the two natural ingredients were used. In Step 2, a difference of one lesion score unit between infected treatment groups was deemed biologically significant.
Materials used were:
6. Sporulated oocysts (in water at titrated dose); prepared as 42 separate tubes containing oocysts of appropriate dilution to provide virulent dose compared to untreated controls.
7. Chickens (50 broilers), arrived at 1 day of age, divided into 5 groups of 10
8. Siliconized microfuge tubes (1.5 ml)
9. Pipettes, pipettors, and pipette tips
10. Test compound
11. Scales or balance for weighing chickens with check weights for scale verification.

The test and measurements were:
6. Day 12 of age, weigh chickens (feed consumption should be measured concurrently with body weight gain)
7. Day 12 of age, begin giving product being tested (compound given to 3 of the 5 groups):
   1. 10 chickens: untreated controls
   2. 10 chickens: untreated controls
   3. 10 chickens: treated at the low dose (40 ppm)
   4. 10 chickens: treated at medium dose (400 ppm)
   5. 10 chickens: treated at high dose (4000 ppm)
8. Day 14 of age, weigh chickens
9. Day 14 of age, orally inoculate chickens with preformed inocula (500,000 oocysts given to each of the chickens in 4 of the 5 groups)
   6. 10 chickens: uninfected and untreated controls
   7. 10 chickens: infected and untreated controls
   8. 10 chickens: infected and treated at the low dose
   9. 10 chickens: infected and treated at the medium dose
   10. 10 chickens: infected and treated at the high dose

All mortality and morbidity, whether resulting from coccidiosis or other causes, should be diagnosed by appropriate tests. Necropsy results should be reported. For diagnosed coccidiosis, wet mount examinations of the ceca should be made and coccidia identified. Body weight, date of death, and gender of any dead birds should be recorded.
Day 21 of age, weigh chickens
Day 21 of age, euthanatize chickens and perform lesion scores (Johnson & Reid, 1970)

| | | |
|---|---|---|
| a. | 0 | No gross lesions |
| b. | +1 | Very few scattered petechiae on the cecal wall; no thickening of the |
| | | cecal walls; normal cecal contents present. |
| c. | +2 | Lesions more numerous with noticeable blood in the cecal contents, |
| | | cecal wall is somewhat thickened; normal cecal contents present. |
| d. | +3 | Large amounts of blood or cecal cores present; cecal walls greatly |
| | | thickened; little, if any, fecal contents in the ceca. |
| e. | +4 | Cecal wall greatly distended with blood or large caseous cores; fecal |
| | | debris lacking or included in cores. Dead birds scored as +4. |

Calculate weight gain for live and dead birds.

| | |
|---|---|
| a. | From Days 12 to 14 of age (This measurement identifies the effects of the |
| | anticoccidial natural product alone). |
| b. | From Days 14 to 21 of age (This measurement assess the effectiveness of |
| | the anticoccidial natural product on infection). |
| c. | From Days 12 to 21 of age (This measurement assesses the effectiveness of |
| | the anticoccidial natural product on infection throughout the experimental |
| | period). |

These studies demonstrated that *Momordica charantia* fruit extract (40 ppm) and *Simarouba amara* bark (400 ppm) treated chickens had final weights, total weight gains, and total percent weight gains that were not significantly different from those in the uninfected control chickens but were significantly higher than those is the uninfected chickens.

| **Group** | **Weight day 7** | | |
|---|---|---|---|
| | **n** | **mean** | **std. dev.** |
| UC | 10 | 716.72 | 88.63 |
| IC | 9 | 607.37 | 99.36 |
| Mom.l (40 ppm) | 10 | 703.65 | 76.34 |
| Mom.m (400 ppm) | 9 | 644.01 | 75.62 |
| Mom.h (4000 ppm) | 9 | 631.01 | 74.51 |
| Sim.l (40 ppm) | 10 | 641.70 | 74.65 |
| Sim.m (400 ppm) | 10 | 681.84 | 62.88 |
| Sim.h (4000 ppm) | 10 | 601.50 | 70.92 |

Improvements in feed efficiency could not be measured statistically (due to the grouping of the chickens), but chickens fed at all levels of *Momordica charantia* and the low and medium levels of *Simarouba amara* had better feed efficiency ratios than the infected control chickens.

| **Group** | **Feed efficiency** | |
|---|---|---|
| | **days -2 to 0** | **days 0 to 7** |
| UC | 1.28 | 1.56 |
| IC | 1.33 | 2.07 |
| Mom.l | 1.21 | 1.59 |
| Mom.m | 1.27 | 1.88 |
| Mom.h | 1.34 | 1.70 |
| Sim.l | 1.32 | 1.77 |
| Sim.m | 1.32 | 1.73 |
| Sim.h | 1.30 | 2.00 |

In the study, the ability of the natural ingredients to decrease lesion scores also was demonstrated. *Momordica* 40 ppm and *Simarouba* 400 ppm both improved the cecal lesion scores by a factor of at least 1.

| **Group** | **Left cecum score** | | **Right cecum score** | **Average cecum score** |
|---|---|---|---|---|
| | **n** | **mean** | **mean** | **mean** |
| UC | 10 | 0.00 | 0.00 | 0.00 |
| IC | 10 | 2.50 | 2.30 | 2.40 |
| Mom.l | 10 | 1.40 | 0.90 | 1.15 |
| Mom.m | 10 | 1.90 | 1.50 | 1.70 |
| Mom.h | 9 | 2.00 | 1.22 | 1.61 |
| Sim.l | 10 | 1.80 | 1.10 | 1.45 |
| Sim.m | 10 | 1.20 | 1.00 | 1.10 |
| Sim.h | 10 | 2.20 | 1.90 | 2.05 |

Statistically significant at p = 0.05, *Momordica* 40 ppm, *Simarouba* 40 ppm, and *Simarouba* 400 ppm improved the average (average of left and right) cecal score. At p = 0.1, *Momordica* 400 ppm and 4000 ppm, improved the average score.

| **Group1** | **Group2** | **p-values for comparison of cecal scores** | | |
|---|---|---|---|---|
| | | **left** | **right** | **average** |
| IC | Mom.l | **0.0199** Mom.l | **0.0024** Mom.l | **0.0066** Mom.l |
| IC | Mom.m | 0.2088 | 0.0772 | 0.0980 |
| IC | Mom.h | 0.2332 | **0.0240** Mom.h | 0.0710 |
| IC | Sim.l | 0.0980 | **0.0079** Sim.l | **0.0333** Sim.l |
| IC | Sim.m | **0.0063** Sim.m | **0.0031** Sim.m | **0.0041** Sim.m |
| IC | Sim.h | 0.4124 | 0.4873 | 0.4200 |

These test results demonstrate that these two natural ingredients, at levels of 40-400 pm in feed, decrease cecal lesion scores, improve weight gain, and maintain feed efficiency in chickens challenged with coccidia. This ability of these two natural ingredients has not been described before.

The use of these two natural ingredients have certain advantages. Given their long history of use in humans, there is extensive information available on the safety of these ingredients, decreasing concerns regarding residues in chickens and chicken products. These natural ingredients also contain compounds not previously used in the control or treatment of coccidian in poultry before, therefore, enabling the control of resistant *Eimeria* strains. Lastly, these natural ingredients contain several compounds, therefore providing a diversity of compounds in the control of coccidia, decreasing the rate at which resistance could emerge.

### Figures

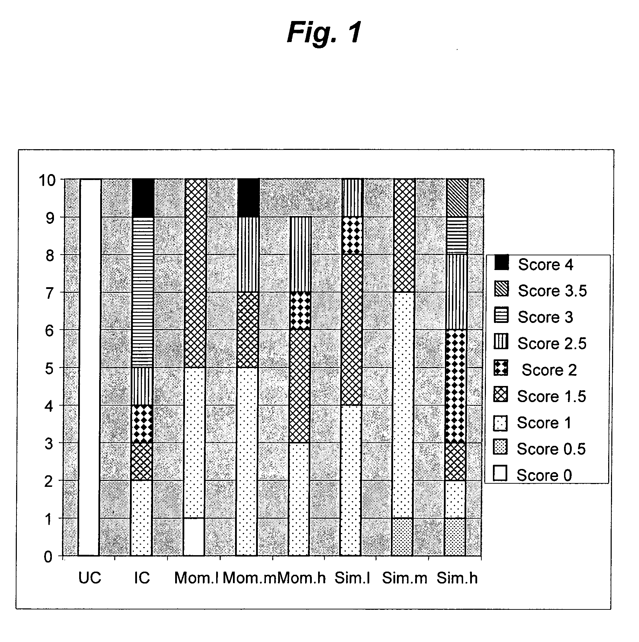

Figure 1 demonstrates that poultry without coccidia have no lesions (Score 0), while 50% of those with untreated infections have lesions scores of 3 or greater. Application of *Simarouba amara* prevents scores of higher than 2.5, while *Momordica charantia* at a rate of 40 ppm maintains scores below 2.

## Claims

1. A veterinary composition for the control of coccidiosis in poultry comprising a coccidiostatically effective amount of *Simarouba amara* dried bark or an extract thereof or of *Momordica charantia* fruit extract or of a coccidiostatically effective amount of a combination of *Simarouba amara* dried bark or an extract thereof and *Momordica charantia* fruit extract together with one or more carriers that are physiologically well-tolerated by birds.

2. The composition according to claim 1 wherein the active ingredient is *Simarouba amara* dried bark or an extract thereof.

3. The composition according to claim 1 wherein the active ingredient is *Momordica charantia* fruit extract.

4. The composition according to claim 1 wherein the active ingredient is a combination of *Simarouba amara* dried bark or an extract thereof and *Momordica charantia* fruit extract.

5. Method for controlling coccidiosis in poultry comprising the administration of a veterinary composition according to any of claims 1 to 4 or of a coccidiostatically effective amount of *Simarouba amara* dried bark or an extract thereof or of *Momordica charantia* fruit extract or of a coccidiostatically effective amount of a combination of *Simarouba amara* dried bark or an extract thereof and *Momordica charantia* fruit extract together with one or more physiologically acceptable carriers to birds suffering from coccidial infections.

6. The method according to claim 5 wherein the active ingredient is *Momordica charantia* fruit extract.

7. The method according to claim 5 wherein the active ingredient is *Simarouba amara* dried bark or an extract therof.

8. The method according to claim 5 wherein the active ingredient is a combination of *Simarouba amara* dried bark or an extract thereof and *Momordica charantia* fruit extract.

9. Use of *Simarouba amara* dried bark or an extract thereof or of *Momordica charantia* fruit extract or of a combination of *Simarouba amara* dried bark or an extract thereof and *Momordica charantia* fruit extract for the preparation of a veterinary composition for the control of coccidiosis in poultry.

10. Use according to claim 9 wherein the active ingredient is *Simarouba amara* dried bark or an extract thereof.

11. Use according to claim 9 wherein the active ingredient is *Momordica charantia* fruit extract.

12. Use according to claim 9 wherein the active ingredient is a combination of *Simarouba amara* dried bark or an extract thereof and *Momordica charantia* fruit extract.

13. Process for producing a veterinary composition for the control of coccidiosis in poultry comprising homogenizing a mixture of one or more carriers that are physiologically acceptable to birds and either a coccidiostatically effective amount of *Simarouba amara* dried bark or an extract thereof or a mixture of one or more physiologically acceptable carriers and *Momordica charantia* fruit extract or a mixture of one or more physiologically acceptable carriers and a coccidiostatically effective amount of a combination of *Simarouba amara* dried bark or an extract thereof and *Momordica charantia* fruit extract.

14. The use of a coccidiostatically effective amount of *Simarouba amara* dried bark or an extract thereof or of *Momordica charantia* fruit extract or of a coccidiostatically effective amount of a combination of *Simarouba amara* dried bark or an extract thereof and *Momordica charantia* fruit extract in a method for treating coccidial infections in poultry.

15. Use according to claim 14 wherein the active ingredient is *Simarouba amara* dried bark or an extract thereof.

16. Use according to claim 14 wherein the active ingredient is *Momordica charantia* fruit extract.

17. Use according to claim 14 wherein the active ingredient is a combination of *Simarouba amara* dried bark or an extract thereof and *Momordica charantia* fruit extract.

18. A poultry feed supplement or medicated poultry feed for the control of coccidiosis in poultry comprising besides ground dry vegetable- and/or animal-based poultry feed, with or without additives such as proteins, vitamins and minerals, a coccidiostatically effective amount of *Simarouba amara* dried bark or an extract thereof or of *Momordica charantia* fruit extract or of a coccidiostatically effective amount of a combination of *Simarouba amara* dried bark or an extract thereof and *Momordica charantia* fruit extract.

19. Poultry feed supplement or medicated poultry feed according to claim 18 wherein the active ingredient is *Simarouba amara* dried bark or an extract thereof.

20. Poultry feed supplement or medicated poultry feed according to claim 18 wherein the active ingredient is *Momordica charantia* fruit extract.

21. Poultry feed supplement or medicated poultry feed according to claim 18 wherein the active ingredient is a combination of *Simarouba amara* dried bark or an extract thereof and *Momordica charantia* fruit extract.

22. Use of a coccidiostatically effective amount of *Simarouba amara* dried bark or an extract thereof or of *Momordica charantia* fruit extract or of a coccidiostatically effective amount of a combination of *Simarouba amara* dried bark or an extract thereof and *Momordica charantia* fruit extract as an additive to dry or wet poultry food or drinking water for the prophylactic or curative treatment of coccidial infections in poultry.

23. Management for preventing the development of resistant *Eimeria* strains that cause coccidiosis in poultry comprising rotation of the administration of a coccidiostatically effective amount of *Simarouba amara* dried bark or an extract thereof and of a coccidiostatically effective amount of *Momordica charantia* fruit extract or alternatively the administration of a coccidiostatically effective amount of a combination of *Simarouba amara* dried bark or an extract thereof and *Momordica charantia* fruit extract together with one or more carriers that are physiologically well-tolerated by birds.

24. Resistance management according to claim 23 wherein during one period of time *Simarouba amara* and the next season *Momordica charantia* is administered.

25. Resistance management according to claim 23 wherein a combination of *Simarouba amara* and *Momordica charantia* is administered.
